# EUROPÄISCHE PATENTSCHRIFT

(11) **EP 0 384 308 B1**
(45) Veröffentlichungstag und Bekanntmachung des Hinweises auf die Patenterteilung: **07.01.1993**
(21) Anmeldenummer: 90103010.6
(22) Anmeldetag: 16.02.1990
(51) Int. Cl.: A61K 35/78

(54) **Extrakt Arzneimittel**
Extract medicament
Extrait, médicament

(30) Priorität: 18.02.1989 DE 3905033
(43) Veröffentlichungstag der Anmeldung: 29.08.1990
(73) Patentinhaber: Wiemann, Wolfram Dr., D-90455 Nürnberg (DE)
(72) Erfinder: Wiemann, Wolfram Dr., D-90455 Nürnberg (DE)
(74) Vertreter: Hafner, Dieter, Dr.rer.nat., Dipl.-Phys.

(56) Entgegenhaltungen:
- CHEMICAL ABSTRACTS, Band 89, Nr. 11, 11. September 1978, Seite 248, Zusammenfassung Nr. 87163q, Columbus, Ohio, US; G. CAIRO VALERA et al.: "A new psychoactive drug: Heisteria olivae (Olacaceae)", & ATTI ACCAD. NAZ. LINCEI, CL. SCI. FIS., MAT. NAT. REND. 1977, 62(3), 363-4

## Beschreibung

Die Erfindung betrifft ein Arzneimittel, sowie die Verwendung eines Extrakts aus der Rinde der Pflanze Heisteria pallida zur Herstellung eines Arzneimittels zur Bekämpfung unterschiedlicher Krankheiten.

Zur Bekämpfung der unterschiedlichsten Krankheiten geht man mehr und mehr auf pflanzliche Mittel über, welche in der Regel unschädlicher sind als synthetische Produkte und sich deshalb einer zunehmenden Beliebtheit bei den Abnehmern im Vergleich zu synthetischen Produkten erfreuen. Besonders bei der Behandlung von rheumatischen Erkrankungen, welche inzwischen zur Volkskrankheit Nummer 1 geworden sind, sind die bisher erhältlichen Naturheilmittel noch nicht wirksam genug, um eine wirklich ernstzunehmende Bekämpfungsmöglichkeit dieser Krankheiten zu bilden.

Es ist daher ein Ziel der Erfindung, ein Arzneimittel bereitzustellen, welches auf breiter Ebene zur Bekämpfung der bedrohlich ansteigenden rheumatischen Erkrankungen sowie zur Vorbeugung dieser gefürchteten Erkrankungen eingesetzt werden kann.

Dieses Ziel wird dadurch gelöst, daß ein Arzneimittel gefunden worden ist, welches einen Extrakt der Pflanze Heisteria pallida enthält.

Die Pflanze Heisteria pallida kommt regelmäßig in tropischen und subtropischen, feuchten Gegenden, wie z. B. Südamerika vor.

Der betreffende Extrakt aus der Rinde dieser Pflanze ist als Mittel zur Bekämpfung von rheumatischen Erkrankungen besonders wirksam. Der Extrakt kann zusätzlich auch als sehr wirksames Mittel zur Vorbeugung dieser Erkrankungen verwendet werden.

Um die Wirksamkeit des Extrakts durch die Herstellungsmethode nicht zu schmälern, wird gemäß einer zweckmäßigen Ausgestaltung des erfindungsgemäßen Extrakts die Extraktion durch Mazeration durchgeführt. Mazeration bedeutet, daß die Ausgangssubstanz mit einem geeeigneten Lösungsmittel zusammengebracht wird, bis nach einer bestimmten Zeit ein Lösungsgleichgewicht erreicht ist, d. h. die löslichen Stoffe aus dem unlöslichen Material getrennt sind. Bei der Mazeration wird folglich auf eine erhöhte Temperatur verzichtet.

Die Extraktion kann jedoch auch durch sog. Perkolation erfolgen, bei der beispielsweise ein Kessel verwendet wird, in den die Rinde eingehängt wird und Lösungsmittel durch diesen Kessel hindurchgepumpt wird.

Vorzugsweise kann weiterhin die Rinde nach dem Zerkleinern gesiebt werden.

Eine besonders wirksame Extraktion erfolgt gemäß einer weiteren Ausgestaltung der Erfindung dadurch, daß als Lösungsmittel ein Alkohol-Wasser-Gemisch verwendet wird. Vorzugsweise umfaßt das Alkohol-Wasser-Gemisch demineralisiertes Wasser sowie 60 - 95%igen Äthylalkohol. Ein besonders wirksamer Extrakt läßt sich gemäß einer weiteren Ausgestaltung der Erfindung dadurch herstellen, daß für 1 kg Rinde etwa 3.000 cm³ - 9.000 cm³, vorzugsweise 6.000 cm³ 90%iger Äthylalkohol sowie 3.000 cm³ - 9.000 cm³, vorzugsweise 6.000 cm³ demineralisiertes Wasser genommen werden.

Um eine vollständige Extraktion zu erreichen, geht gemäß einer weiteren Ausgestaltung des erfindungsgemäßen Extrakts die Mazeration über einen Zeitraum von 0,2 bis über 5 Wochen.

Die Extraktion kann dadurch verbessert werden, daß die gleichen Rindenstückchen zur Bildung mehrerer Extrakte mehrmals in frischem Lösungsmittel mazeriert und die verschiedenen Extrakte anschließend miteinander vermischt werden. Hierdurch läßt sich der Extraktionsgrad steigern und die Extraktionszeit verkürzen.

Eine gemäß einer weiteren Ausgestaltung des erfindungsgemäßen Arzneimittels besonders wirksame Zusammensetzung ist dadurch gekennzeichnet, daß 100 g Arzneimittel eine Extraktionsmenge an Wirkstoff enthalten, die einer Menge von 0,5 g - 20 g Rinde der Pflanze Heisteria pallida entspricht. Die Extraktionsmenge kann in zweckmäßiger Weise einer Menge von 0,05 g - 20,0 g, vorzugsweise 0,5 g - 10,0 g Rinde der Pflanze Heisteria pallida entsprechen. Eine besonders bevorzugte Zusammensetzung des Arzneimittels ist dadurch gekennzeichnet, daß die Extraktionsmenge einer Menge von etwa 1,0 g - 6,0 g, vorzugsweise 1,0 g - 2,0 g Rinde der Pflanze Heisteria pallida entspricht.

Gemäß einer weiteren Ausgestaltung des erfindungsgemäßen Arzneimittels enthält das Arzneimittel neben dem Extrakt der Rinde der Pflanze Heisteria pallida zusätzlich einen Extrakt von Radix harpagophytum procumbens (Teufelskralle) und/oder ein Extrakt von Cortex salicis (Weidenrinde und/oder ein Extrakt von Folia Betulae (Birkenblätter). Bei der Teufelskralle handelt es sich um eine aus Afrika stammende Pflanze.

Zur Gewährleistung der gewünschten Wirkungen liegen, gemäß einer weiteren Ausgestaltung des erfindungsgemäßen Arzneimittels, die Anteile der Extrakte zu je gleichen Teilen vor.

Um den etwas bitteren Eigengeschmack des Extrakts zu vermeiden, ist eine weitere zweckmäßige Form des Arzneimittels durch einen Zusatz eines Geschmacksbildners gekennzeichnet. Als Geschmacksbildner kann hierbei zweckmäßigerweise Zuckersirup, oder bei Diabetikern, Sorbit zugegeben werden. Da das Arzneimittel gemäß einer weiteren Ausgestaltung in Form von Tabletten, Tropfen, Dragees, Tonikum, Injektionen oder als Tee verabreichbar ist, kann das Mittel in der jeweils für den betreffenden Patienten geeignetsten Weise verabreicht werden.

Der erfindungsgemäße Extrakt kann in vorteilhafter Weise sowohl zur Behandlung als auch zur Vorbeugung von Erkrankungen aus dem rheumatioden Formenkreis eingesetzt werden. Insbesondere eignet sich der Extrakt zur Behandlung von Rheuma, Arthritis, entzündlicher Arthrose, zur Behandlung von Bewegungseinschränkungen bis hin zu Lähmungen, zur Behandlung von Extremitätenentzündungen, zur Behandlung von Wirbelsäulenentzündungen, zur Behandlung entzündlicher Erkrankungen im Schulter-Arm-Bereich sowie der Muskulatur, Sinusitis, zur Behandlung von Gicht, aber auch überraschenderweise zur Behandlung von fiebrigen Erkrankungen, insbesondere Malaria bzw. Sumpffieber. Ferner kann der erfindungsgemäße Extrakt als entwässern- des Mittel, als Mittel zur Behandlung von Diabetes sowie als Aphrodisiacum eingesetzt werden.

### HERSTELLUNG

### Beispiel 1:

Im Folgenden wird lediglich beispielhaft die Herstellung des erfindungsgemäßen Extrakts bzw. Arzneimittels näher erläutert. Man geht beispielsweise von 1 kg Rinde der Stammpflanze Heisteria pallida aus. Diese Rinde wird mittels einer geeigneten Zerkleinerungsvorrichtung (Mühle) in eine gewünschte Fraktion zerkleinert und anschließend gesiebt. Bei der Zerkleinerung ist darauf zu achten, daß die Fraktion nicht zu fein zerkleinert wird, da es dann zu einer Verklumpung der Rindenbestandteile kommen kann. Der Siebrückstand wird in ein Lösungsmittel eingelegt. Als Lösungsmittel wird ein Alkohol-Wasser-Gemisch verwendet, welches beispielsweise insgesamt 6.000 cm³ 90%igen Äthylalkohol sowie 6.000 cm³ demineralisiertes Wasser auf 1 kg Rinde umfaßt.

In diesem Lösungsmittel erfolgt die Mazeration, d. h. das Auslösen der betreffenden Wirkstoffanteile aus der Rinde solange, bis ein Lösungsgleichgewicht erreicht ist. Anstelle der Mazeration kann auch eine Perkolation durchgeführt werden.

Die betreffenden Rindenstückchen werden mehrmals in neues frisches Lösungsmittel, bestehend aus Alkohol und Wasser, eingelegt, wobei dieser Vorgang vorteilhafterweise sechsmal durchgeführt werden kann. Die derart erhaltenen Extrakte unterschiedlicher Konzentration müssen anschließend untereinander gut gemischt werden. Anschließend ist es möglich, mittels üblicher Verfahren, Tabletten, Tropfen, Dragees, ein Tonikum, eine Injektionslösung oder dergleichen herzustellen. Man kann die Rinde selbst jedoch auch zur Bereitung von verwenden.

Zur Verbesserung der Einnahme, d. h. zur Beseitigung des etwas bitteren Eigengeschmacks des Extrakt, kann beispielsweise Sorbit in einem Verhältnis von Extrakt:Zuckersirup bzw. Sorbit von 80:20 zugegeben werden.

Das betreffende Arzneimittel sollte in Form eines Tonikums bzw. in Tropfenform einen Auszug von 1,0 g - 2,0 g bzw. 5,0 g - 7,0 g Rinde auf 100 g Arzneimittel aufweisen.

### Beispiel 2:

Eine weitere Rezeptur umfaßt 140 cm³ Extrakt Heisteria pallida, 70 g Zuckersirup, 200 g Ansatzwein, wobei der Rest mit Wasser bis auf 700 cm³ aufgefüllt wird.

Weitere vorteilhafte Wirkungen ergeben sich durch Zusatz eines Extrakts von Radix harpagophytum procumbens (Teufelskralle). Bei der Teufelskralle handelt es sich um eine afrikanische Pflanze. Die Anteile des Extrakts aus Heisteria pallida sowie der aus Teufelskralle können in etwa gleich sein.

Zusätzlich zum Extrakt der Teufelskralle oder anstelle davon können auch Extrakte von Cortex salicis (Weidenrinde) und Folia betulae (Birkenblätter) vorzugsweise zu jeweils gleichen Teilen, zugegeben werden.

Das erfindungsgemäße Arzneimittel bzw. Extrakt kann bei den verschiedensten Erkrankungen rheumatischer Art, z. B. entzündlichen Erkrankungen im Schulter-Arm-Bereich, der Muskulatur der Gelenke, der Wirbelsäule, Versteifungen von Extremitäten bis hin zu Lähmungen, Arthritis und Arthrose, soweit entzündlich, sowie generell bei Erkrankungen entzündlichen Ursprungs angewendet werden.

Die Anwendung des Arzneimittels ist nicht auf den akuten Zustand beschränkt, sondern er stellt auch ein wirksames Mittel zur Vorbeugung dieser gefürchteten Erkrankungen dar.

Auch zur Behandlung von Gicht ist das erfindungsgemäße Mittel besonders geeignet.

## Patentansprüche

1. Arzneimittel,
**dadurch gekennzeichnet,**
daß es einen Extrakt aus der Rinde der Pflanze Heisteria pallida enthält.

2. Arzneimittel nach Anspruch 1 ,
**dadurch gekennzeichnet,**
daß es einen Extrakt aus Radix harpagophytum procumbens (Teufelskralle) Zusätzlich enthält.

3. Arzneimittel nach den Ansprüchen 1 oder 2,
**dadurch gekennzeichnet,**
daß es einen Extrakt aus Folia betulae (Birkenblätter) zusätzlich enthält.

4. Arzneimittel nach den Ansprüchen 1 - 3,
**dadurch gekennzeichnet,**
daß es einen Extrakt aus Cortex salicis (Weidenrinde) zusätzlich enthält.

5. Arzneimittel nach den Ansprüchen 2 - 4,
**dadurch gekennzeichnet,**
daß im Arzneimittel die einzelnen Extrakten in etwa zu gleichen Teilen vorliegen.

6. Verwendung eines Extrakts aus der Rinde der Pflanze Heisteria pallida zur Herstellung eines Arzneimittels zur Behandlung von Erkrankungen aus dem rheumatoiden Formenkreis.

7. Verwendung eines Extrakts aus der Rinde der Pflanze Heisteria pallida zur Herstellung eines Arzneimittels zur Behandlung von infektiösen, fiebrigen Erkrankungen, insbesondere Malaria.

## Claims

1. Pharmaceutical product
characterized in that
it contains an extract from the bark of the plant *Heisteria pallida*.

2. Pharmaceutical product, in accordance with claim 1,
characterized in that
it also contains an extract from Radix *harpagophytum procumbens*.

3. Pharmaceutical product, in accordance with either of claims 1 or 2,
characterized in that
it also contains an extract from birch-leaves *(folia betulae).*

4. Pharmaceutical product, in accordance with claims 1 to 3,
characterized in that
it also contains an extract from willow-bark *(cortex salicis)*.

5. Pharmaceutical product in accordance with claims 2 to 4,
wherein
the separate extracts are present in approximately equal proportions.

6. Utilization of an extract from the bark of the plant *Heisteria pallida* in the production of a pharmaceutical product for the treatment of sicknesses of the rheumatoid kind.

7. Utilization of an extract from the bark of the plant *Heisteria pallida* in the production of a pharmaceutical product for the treatment of infectious, febrile sicknesses, malaria in particular.

## Revendications

1. Médicament, caractérisé en ce qu'il contient un extrait de l'écorce de la plante Heisteria pallida.

2. Médicament suivant la revendication 1, caractérisé en ce qu'il contient en sus un extrait de Radix harpagophytum procumbens (griffe du diable).

3. Médicament suivant la revendication 1 ou 2, caractérisé en ce qu'il contient en sus un extrait de Folia betulae (feuilles de bouleau).

4. Médicament suivant l'une des revendications 1 à 3, caractérisé en ce qu'il contient en sus un extrait de Cortex salicis (écorce de saule).

5. Médicament suivant l'une des revendications 2 à 4, caractérisé en ce que les différents extraits sont présents dans ce médicament dans des proportions pratiquement identiques.

6. Utilisation d'un extrait de l'écorce de la plante Heisteria pallida pour élaborer un médicament servant au traitement de maladies du domine des formes à symptômes rhumatoïdes.

7. Utilisation d'un extrait de l'écorce de la plante Heisteria pallida pour élaborer un médicament servant au traitement de maladies infectieuses fébrigènes, notamment le paludisme.
